# EUROPEAN PATENT SPECIFICATION

(11) **EP 3 929 178 B1**
(45) Date of publication and mention of the grant of the patent: **31.12.2025**
(21) Application number: 19935712.0
(22) Date of filing: 12.11.2019
(51) Int. Cl.: C07C 319/28, C07C 323/52, A61K 31/22, A61P 31/04

(54) **CRYSTAL FORM OF VALNEMULIN HYDROCHLORIDE HYDRATE, PREPARATION METHOD THEREFOR, AND PHARMACEUTICAL COMPOSITION CONTAINING CRYSTAL FORM**
KRISTALLFORM VON VALNEMULIN-HYDROCHLORIDHYDRAT, VERFAHREN ZU IHRER HERSTELLUNG UND DIESE ENTHALTENDE PHARMAZEUTISCHE ZUSAMMENSETZUNG
FORME CRISTALLINE D'UN HYDRATE D'HYDROCHLORURE DE VALNEMULIN, SON PROCÉDÉ DE PRÉPARATION ET COMPOSITION PHARMACEUTIQUE CONTENANT LA FORME CRISTALLINE

(30) Priority: 23.06.2019 CN 201910545895
(43) Date of publication of application: 29.12.2021
(73) Proprietor: Hubei Longxiang Pharmaceutical Co., Ltd., Hubei 435406 (CN); Ringpu (Tianjin) Bio-Pharmacy Co., Ltd., Dongli Development Zone Tianjin 300300 (CN)
(72) Inventor: LI, Yaling, Tianjin 300300 (CN); WU, Yanzi, Tianjin 300300 (CN); WANG, Biao, Tianjin 300300 (CN); LI, Qifeng, Tianjin 300300 (CN); XIA, Xuelin, Tianjin 300300 (CN); LIU, Ailing, Tianjin 300300 (CN); LI, Shoujun, Tianjin 300300 (CN); FU, Chunxiang, Tianjin 300300 (CN)
(74) Representative: Ricker, Mathias
(86) International application number: PCT/CN2019/117679
(87) International publication number: WO 2020/258660

(56) References cited:
- CN-A- 104 876 841
- CN-A- 110 294 697
- OUYANG JINBO, NA BING, ZHOU LIMIN, XIAO SAIJIN, XIONG GUOXUAN, JIN TIANXIANG: "Crystal structures and phase transformation of two novel solvates of valnemulin hydrochloride", CRYSTENGCOMM, vol. 20, no. 5, 7 February 2018 (2018-02-07), pages 563 - 569, XP055774588, DOI: 10.1039/C7CE01524K
- OUYANG JINBO; NA BING; LIU ZHIRONG; ZHOU LIMIN; HAO HONGXUN: "Determination of Solubility and Nucleation Kinetics of Valnemulin Hydrochloride Solvate", JOURNAL OF SOLUTION CHEMISTRY, PLENUM PUBLISHING, NEW YORK, NY,, US, vol. 48, no. 4, 27 March 2019 (2019-03-27), US, pages 413 - 426, XP036761817, ISSN: 0095-9782, DOI: 10.1007/s10953-019-00861-7

## Description

### TECHNICAL FIELD

The present application belongs to the technical field of veterinary drugs and chemical engineering crystallization, and specifically relates to a crystal form of a valnemulin hydrochloride hydrate, a preparation method thereof, and a pharmaceutical composition containing the crystal form.

### BACKGROUND

Valnemulin hydrochloride has a molecular formula of C₃₁H₅₂N₂O₅S·HCl and a molecular weight of 601.29. It is a white powder with pungent odor. It has the following structural formula:

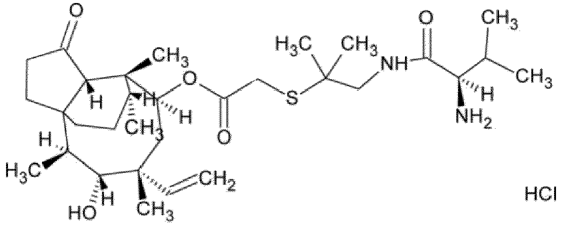

Valnemulin hydrochloride is a next-generation pleuromutilin-like semi-synthetic antibiotics, and belongs to the class of diterpenes. It is an animal-specific antibiotic mainly used for the prevention and treatment of mycoplasma and gram-positive bacterial infections in pigs, cattle, sheep and poultry. It is mainly concentrated in lungs and is an ideal drug for the treatment of pulmonary diseases caused by various mycoplasmas. Valnemulin interacts with the 50S subunit on the ribosome of pathogenic microorganisms, thereby inhibiting protein synthesis in pathogenic microorganisms and leading to death of the pathogenic microorganisms. Valnemulin hydrochloride crystals are difficult to prepare by ordinary crystallization methods. Therefore, valnemulin hydrochloride drugs currently produced and sold at home and abroad are all in an amorphous state.

For solid drugs, different solid forms will have an important impact on physical and chemical properties such as stability, solubility, dissolution rate, bulk density and fluidity, resulting in different efficacy and bioavailability during use. Therefore, for solid pharmaceutical products, the development of different solid forms is more conducive to choosing a suitable crystal form according to the characteristics of the pharmaceutical preparation. For valnemulin hydrochloride products, compared with crystalline products, the amorphous products have problems such as poor stability, easy hygroscopicity, poor appearance, poor fluidity, and difficult storage. At the same time, aqueous solutions of amorphous valnemulin hydrochloride products are unstable during preparation process. Experiments have shown that 11.6% loss is caused when granulated at 75 °C. Therefore, there is an urgent need to develop a stable valnemulin hydrochloride crystal product to meet market demand for veterinary drugs. However, through literature search, it is found that most of the literature is about the synthesis and preparation of valnemulin hydrochloride products, and the products obtained are all amorphous. There are very few reports on the crystal form of valnemulin hydrochloride and preparation method thereof.

Chinese patent CN108892629A discloses a preparation method of valnemulin hydrochloride methyl tert-butyl ether solvent, and a solvent-free compound prepared from a solvent-containing compound. However, the solvent-free compound has a bulk density of only 0.305 g/ml, which is not improved compared with the bulk density of the amorphous compound. Moreover, it is not indicated whether the obtained solvent-free compound is a crystal. Therefore, there is still a need in the field to obtain crystals of valnemulin hydrochloride with high purity, high bulk density and good palatability suitable for pharmaceutical preparation.

Patent CN104876841B reports a cooling-ultrasonic crystallization method for preparing valnemulin hydrochloride, which affords a crystalline compound of valnemulin hydrochloride showing characteristic peaks at 2θ of 8.5±0.1, 10.6±0.1, 10.9±0.1, 11.2±0.1, 12.1±0.1, 12.6±0.1, 15.0±0.1, 17.8±0.1, 18.4±0.1, 19.3±0.1, 20.2±0.1, 20.8±0.1, 25.8±0.1, 27.7±0.1, 28.4±0.1, 29±0.1. However, the crystallization process in this invention is not easy to industrialize, and although the crystalline compound has a certain improvement in hygroscopicity, the problem of hygroscopicity still exists.

Ouyang Jinbo et al. (2018) CrystEngComm, vol. 20, no. 5, 7, pages 563-569 published Crystal structures and phase transformation of two novel solvates of valnemulin hydrochloride. Ouyang Jinbo et al. (2019) Journal of solution chemistry, plenum publishing, vol. 48, no. 4, 27, pages 413-426 published Determination of Solubility and Nucleation Kinetics of Valnemulin Hydrochloride Solvate.

Therefore, there is a greater need in the art to obtain valnemulin hydrochloride crystals that do not contain organic solvents, have high purity, do not have hygroscopicity, and have better stability to meet market demand. At the same time, it is expected in the art to further broaden the raw material library for preparation selection, and design new dosage forms of drugs with improved characteristics, so that the drugs may be more widely used in dosage forms such as oral and injection preparations.

### SUMMARY

The following is a summary of the topics detailed herein. This summary is not intended to limit the scope of protection of the claims.

The present application provides a crystal form of a valnemulin hydrochloride hydrate, a preparation method thereof, and a pharmaceutical composition containing the crystal form. The obtained hydrate crystal form has a large bulk density, no hygroscopicity, and good thermal stability. The hydrate crystal form and the pharmaceutical composition containing the crystal form are widely used in pharmaceutical preparations, and can be used to prepare dosage forms such as soluble powders, sustained-release granules, injections, oral liquids, ampoules, etc., with broad clinical application prospects.

The purpose of the present application is achieved through the following technical solutions:
The first aspect of the present application provides a crystal form of a valnemulin hydrochloride hydrate, which shows characteristic peaks in spectrum at 2θ diffraction angles of 9.2±0.2°, 9.5 ± 0.2°, 10.3 ± 0.2°, 11.8 ± 0.2°, 12.3 ± 0.2°, 12.8 ± 0.2°, 15.1 ± 0.2°, 17.5 ± 0.2°, 18.2 ± 0.2° as measured by X-ray powder using CuKa rays as characteristic;
wherein a moisture content (mass content) of the crystal form of the valnemulin hydrochloride hydrate is 0.1% to 7%.

The crystal form of the valnemulin hydrochloride hydrate further comprises characteristic peaks in spectrum at one or more of 2θ diffraction angles of 4.4±0.2°, 12.6±0.2°, 14.4±0.2°, 14.6 ±0.2°, 16.5±0.2°, 17.9±0.2°, 18.7±0.2°, 25.8±0.2°, 28.7±0.2° as measured by X-ray powder using CuKa rays as characteristic.

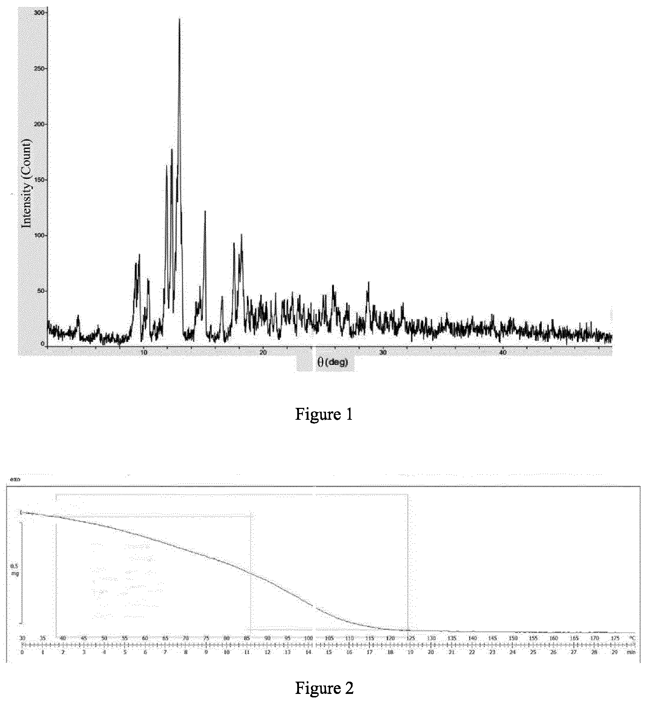

The X-ray powder diffraction pattern of the crystal form of valnemulin hydrochloride hydrate is shown in FIG. 1.

The crystal form of the valnemulin hydrochloride hydrate has an endothermic peak at 120±5 °C in the DSC spectrum.

The second aspect of the present application provides a preparation method of the crystal form of the valnemulin hydrochloride hydrate, which specifically includes the following steps:
(1) thermally-dissolving valnemulin hydrochloride in a mixture of organic solvent/purified water at 20 °C to 60 °C ;
(2) stirring under heat preservation for 0.5 h to 1 h, and cooling at -5 °C to 15 °C to precipitate a crystal from the mixed solution;
(3) filtering to obtain filter cake A;
(4) drying the filter cake A under vacuum at 20 °C to 40 °C to obtain filter cake B;
(5) stirring and equilibrating the filter cake A obtained in step (3) in a poor solvent for 6 h to 20 h, filtering, and drying under vacuum at 20 °C to 60 °C to obtain a hydrate crystal; or, humidifing the filter cake B obtained in step (4) at a humidity of 65% to 95% for 12 h to 48 h to obtain a hydrate crystal.

The organic solvent is selected from one or more of ester, ketone, alcohol, furan or acetonitrile solvents.

Preferably, the organic solvent is selected from one or more of ethyl acetate, acetone, isopropanol, n-butanol, tert-butanol, methanol, ethanol, tetrahydrofuran or acetonitrile.

The poor solvent is selected from one or more of alkane solvents, aromatic hydrocarbons, ether solvents or water.

Preferably, the poor solvent is selected from one or more of n-hexane, cyclohexane, n-heptane, diethyl ether, dimethyl ether, diisopropyl ether, methyl tert-butyl ether, toluene or water.

The weight-volume ratio of valnemulin hydrochloride as an active pharmaceutical ingredient and organic solvent/purified water is 1:1 to 1:10; the weight-volume ratio of valnemulin hydrochloride as an active pharmaceutical ingredient and the poor solvent is 1:2 to 1:15.

The volume ratio of the organic solvent and purified water is 2:4 to 3:2.

The third aspect of the present application provides a pharmaceutical composition containing the crystal form of the valnemulin hydrochloride hydrate of the present application.

The pharmaceutical composition is a mixture of the crystal form of the valnemulin hydrochloride hydrate and another crystal form of valnemulin hydrochloride.

Or, the pharmaceutical composition further contains one or more pharmaceutically acceptable carriers and/or excipients.

The fourth aspect of the present application provides use of the crystal form of the valnemulin hydrochloride hydrate or the pharmaceutical composition of the present application in the preparation of pharmaceutical preparations.

The pharmaceutical preparations can be prepared into dosage forms including tablets, soluble powders, injections, oral liquids, sustained-release granules, other granules, freeze-dried powders, ampoules, solid dispersions, pellets, and suspensions.

The beneficial effects of the present application are as follows:
(1) The present application uses a conventional crystallization reactor kettle for crystallization, with simple operating steps and preparation processes, stable and easy-to-repeat preparation processes, high yields, easy industrial production, and significant practicability.
(2) The crystal form of the valnemulin hydrochloride hydrate has a large bulk density, no hygroscopicity, strong fluidity, good stability, and no pungent odor.
(3) The crystal form of the valnemulin hydrochloride hydrate and pharmaceutical composition thereof can be used in the preparation of a variety of pharmaceutical preparations.

After reading and understanding the drawings and detailed description, other aspects can be understood.

### BRIEF DESCRIPTION OF DRAWINGS

In order to more clearly describe the technical solutions in the examples of the present application or related technologies, the accompanying drawings that need to be used in the examples will be briefly introduced below.
FIG. 1 is an X-ray diffraction pattern of the crystal form of valnemulin hydrochloride hydrate described in Example 1 of the present application.
FIG. 2 is a TGA spectrum of the crystal form of the valnemulin hydrochloride hydrate described in Example 1 of the present application.

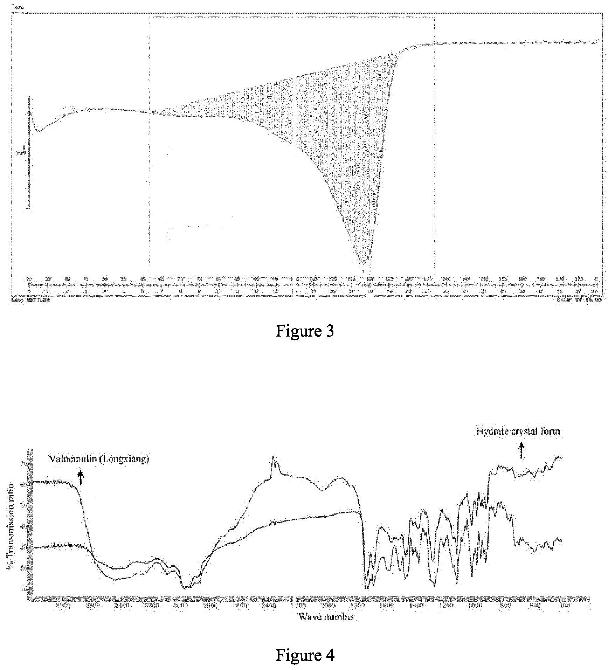

FIG. 3 is a DSC spectrum of the crystal form of the valnemulin hydrochloride hydrate described in Example 1 of the present application.
FIG. 4 shows comparison of IR spectrum between the valnemulin hydrochloride hydrate crystal described in Example 1 of the present application and an amorphous raw material.

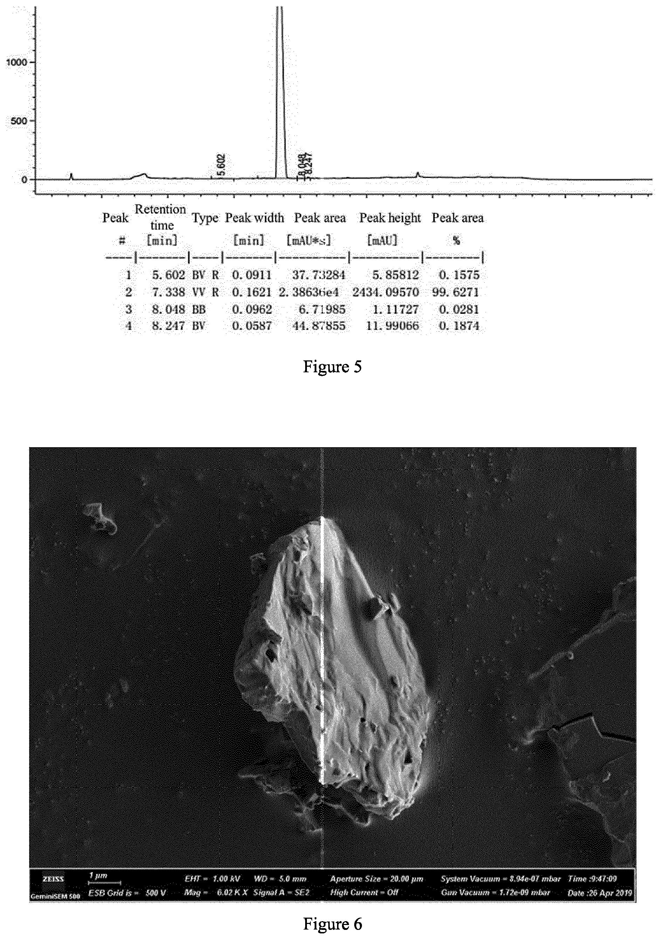

FIG. 5 is an HPLC chromatogram of the crystal form of the valnemulin hydrochloride hydrate described in Example 1 of the present application.
FIG. 6 is a scanning electron micrograph of the crystal form of the valnemulin hydrochloride hydrate prepared of the present application.

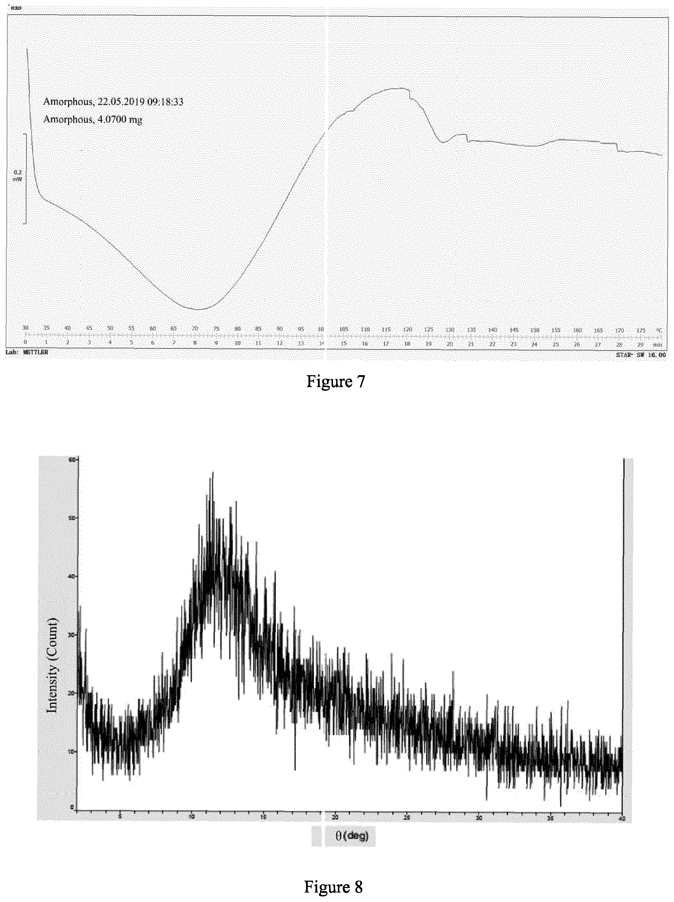

FIG. 7 is a DSC spectrum of the amorphous valnemulin hydrochloride raw material.
FIG. 8 is an XRD pattern of the amorphous valnemulin hydrochloride raw material.

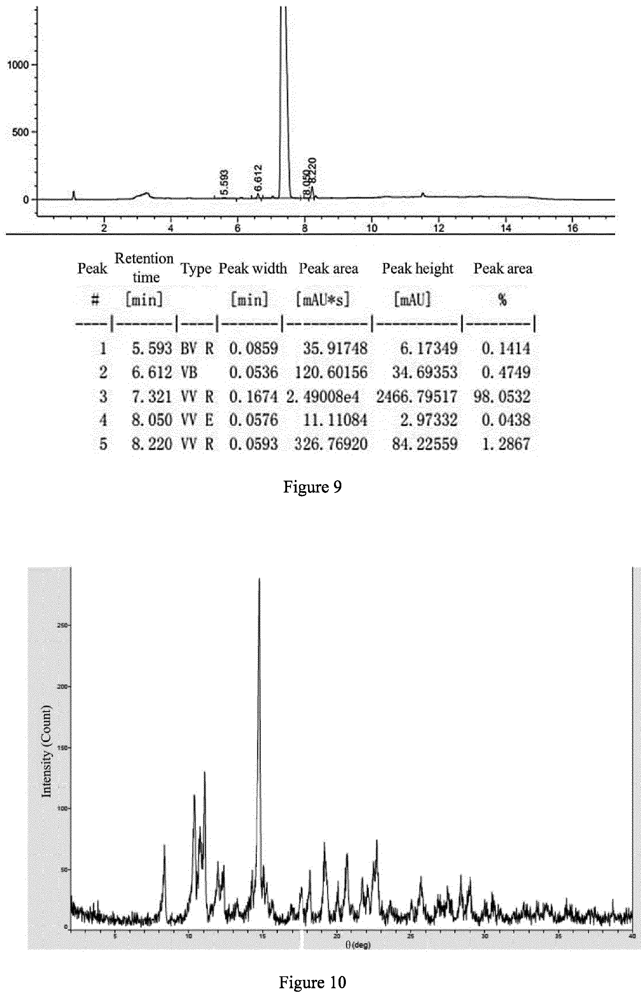

FIG. 9 is an HPLC chromatogram of the amorphous valnemulin hydrochloride raw material.
FIG. 10 is an XRD pattern of the crystal form of patent CN104876841B.

### DETAILED DESCRIPTION

In order to further understand the present application, the preferred embodiments of the present application are described below in conjunction with examples, but it should be understood that these descriptions are only to further illustrate the features and advantages of the present application, and not to limit the claims of the present application.

Raw materials and general test methods:
The valnemulin hydrochloride used in the examples as an active pharmaceutical ingredient was purchased from Hubei Longxiang Pharmaceutical.

X-Ray Powder Diffraction (XRD) instrument: Japan Rigaku D/Max-2500 type; radiation source: copper target, scanned at room temperature; scanning range: 2.0° to 50.0°; step size: 0.02°; and scanning rate: 8°/min.

Differential Scanning Calorimetry (DSC) instrument: Switzerland METTLER TOLEDO DSC1 type, within the range of 30 °C to 180 °C; heating rate: 5 °C/min, with nitrogen protection.

Thermal Gravimetric Analyzer (TGA) instrument: Switzerland METTLER TOLEDO TGA1 type, within the range of 30 °C to 180 °C; heating rate: 5 °C/min, with nitrogen protection.

Conditions for purity detection by High Performance Liquid Chromatography (HPLC): octadecylsilane-bonded silica gel was used as filler (150 mm×4.6 mm, 3 µm); mobile phase A was phosphate buffer (pH 2.5); mobile phase B was phosphate buffer (pH 2.5)-acetonitrile (25:75); the flow rate was 1.5 ml per minute; the column temperature was 50 °C; and the detection wavelength was 200 nm. Linear gradient elution was performed according to the following table.

| Time (min) | Mobile phase A (%) | Mobile phase B (%) |
|---|---|---|
| 0 | 95 | 5 |
| 2 | 55 | 45 |
| 4.5 | 50 | 50 |
| 5.5 | 35 | 65 |
| 6.85 | 35 | 65 |
| 10 | 0 | 100 |
| 13 | 0 | 100 |
| 14 | 95 | 5 |
| 20 | 95 | 5 |

### Example 1

10 g of valnemulin hydrochloride as the active pharmaceutical ingredient was taken, 30 ml of methanol was added, then 20 ml of purified water was added. The mixture was heated to a temperature of 40 °C to dissolve, stirred under heat preservation for 1 hour, then slowly cooled to a temperature of 5 °C, stirred and crystalized for 4 h, and filtered. The filter cake was dissolved in 100 ml of n-hexane and stirred for 6 h, and filtered. The filter cake was dried under vacuum at 30 °C to obtain 9.5 g of a valnemulin hydrochloride hydrate, with a yield of 95%, a normalized purity of 99.63%, and a moisture content of 6.9%.

### Example 2

10 g of valnemulin hydrochloride as the active pharmaceutical ingredient was taken, 40 ml of ethyl acetate was added, then 50 ml of purified water was added. The mixture was heated to a temperature of 60 °C to dissolve, stirred under heat preservation for 1 hour, then slowly cooled to a temperature of 10 °C, stirred and crystalized for 8 h, and filtered. The filter cake was dissolved in 150 ml of n-heptane and stirred for 8 h, and filtered. The filter cake was dried under vacuum at 40 °C to obtain 9.2 g of a valnemulin hydrochloride hydrate, with a weight yield of 92%, a purity of 98.8%, and a moisture content of 6.5%.

### Example 3

10 g of valnemulin hydrochloride as the active pharmaceutical ingredient was taken, 50 ml of ethanol was added, then 35 ml of purified water was added. The mixture was heated to a temperature of 55 °C to dissolve, stirred under heat preservation for 1 hour, then slowly cooled to a temperature of -5 °C, stirred and crystalized for 10 h, and filtered. The filter cake was dried under vacuum at 35 °C for 12 hours and placed over a saturated potassium nitrate aqueous solution (92.5% RH) for 16 hours, to afford a crystal form of valnemulin hydrochloride 2.5-hydrate, with a moisture content of 6.8%.

The crystal form of valnemulin hydrochloride 2.5-hydrate was dried at a temperature of 40 °C for 20 h to constant weight, to afford a crystal form of valnemulin hydrochloride 1.5-hydrate, with a moisture content of 4.6%.

The crystal form of valnemulin hydrochloride 1.5-hydrate was continued to be dried at a temperature of 60 °C for 20 h to constant weight, to afford 8.9 g of a crystal form of valnemulin hydrochloride 1.25-hydrate, with a yield of 89%, an HPLC purity of 98.9%, and a moisture content of 3.7%.

### Example 4

10 g of valnemulin hydrochloride as the active pharmaceutical ingredient was taken, 35 ml of tert-butanol was added, then 35 ml of purified water was added. The mixture was heated to a temperature of 56 °C to dissolve, stirred under heat preservation for 1 hour, then slowly cooled to a temperature of 8 °C, stirred and crystalized for 9 h, and filtered. The filter cake was dissolved in 80 ml of methyl tert-butyl ether and stirred for 20 h, and filtered. The filter cake was dried under vacuum at 38 °C to obtain 9.8 g of a valnemulin hydrochloride hydrate, with a yield of 98%, a purity of 99.2%, and a moisture content of 6.5%.

### Example 5

10 g of valnemulin hydrochloride as the active pharmaceutical ingredient was taken, 40 ml of acetone was added, then 32 ml of purified water was added. The mixture was heated to a temperature of 45 °C to dissolve, stirred under heat preservation for 0.5 hour, then slowly cooled to a temperature of 0 °C, stirred and crystalized for 11 h, and filtered. The filter cake was dissolved in 70 ml of diisopropyl ether and stirred for 17 h, and filtered. The filter cake was dried under vacuum at 40 °C to obtain 9.5 g of a valnemulin hydrochloride hydrate, with a yield of 95%, a purity of 99.4%, and a moisture content of 6.4%.

### Example 6

10 g of valnemulin hydrochloride as the active pharmaceutical ingredient was taken, 30 ml of ethanol and 5 ml of n-butyl alcohol were added, then 35 ml of purified water was added. The mixture was heated to a temperature of 52 °C to dissolve, stirred under heat preservation for 0.5 hour, then slowly cooled to 12°C, stirred and crystalized for 8 h, and filtered. The filter cake was dried under vacuum at 30 °C for 12 hours and placed over a saturated sodium chloride aqueous solution (75% RH) for 32 hours, to afford a crystal form of valnemulin hydrochloride 2.5-hydrate, with a moisture content of 6.8%.

The crystal form of valnemulin hydrochloride 2.5-hydrate was dried at a temperature of 45 °C for 20 h to a constant weight, to afford a crystal form of valnemulin hydrochloride 1.5-hydrate, with a moisture content of 4.5%.

The crystal form of valnemulin hydrochloride 1.5-hydrate was continued to be dried at a temperature of 70 °C for 12 h to constant weight, to afford 8.84 g of a crystal form of valnemulin hydrochloride monohydrate, with a yield of 88.4%, an HPLC purity of 99.2%, and a moisture content of 2.9%.

### Example 7

10 g of valnemulin hydrochloride as the active pharmaceutical ingredient was taken, 40 ml of acetonitrile was added, then 44 ml of purified water was added. The mixture was heated to a temperature of 50 °C to dissolve, stirred under heat preservation for 0.5 hour, then slowly cooled to a temperature of -2 °C, stirred and crystalized for 12 h, and filtered. The filter cake was placed in 20 ml of purified water and stirred for 19 h, and filtered. The filter cake was dried under vacuum at 40 °C to obtain 9.5 g of a valnemulin hydrochloride hydrate, with a yield of 92%, a purity of 99.5%, and a moisture content of 6.7%.

### Conclusion:

The X-ray powder diffraction pattern of the crystal form of the valnemulin hydrochloride hydrate in Example 1 had characteristic peaks at diffraction angle 2θ = 9.2°, 9.5°, 10.3°, 11.8°, 12.3°, 12.6°, 12.8°, 14.4°, 14.6°, 15.1°, 16.5°, 17.5°, 17.9°, 18.2°, 25.8°, 28.7°. The DSC spectrum had a characteristic peak at 119.5°C; the melting point of the hydrate product was 119.5°C, which was 50°C higher than the temperature of the amorphous desolventized product. The TGA spectrum showed that the weight loss was 6.4%. Compared with the original amorphous product, the hydrate product had no pungent odor and no hygroscopicity, large bulk density and enhanced stability.

The X-ray powder diffraction pattern of the crystal form of the valnemulin hydrochloride hydrate in Example 2 had characteristic peaks at diffraction angle 2θ = 9.1°, 9.5°, 10.2°, 11.7°, 12.2°, 12.7°, 12.9°, 14.3°, 14.5°, 15.2°, 16.4°, 17.4°, 17.8°, 18.2°, 25.8°, 28.8°. The DSC spectrum had a characteristic peak at 120.1°C; the melting point of the hydrate product was 119.1°C, which was 50°C higher than the temperature of the amorphous desolventized product. Compared with the original amorphous product, the hydrate product had no pungent odor and no hygroscopicity.

The X-ray powder diffraction pattern of the crystal form of the valnemulin hydrochloride hydrate in Example 3 had characteristic peaks at diffraction angle 2θ = 9.2°, 9.4°, 10.3°, 11.7°, 12.3°, 12.5°, 12.7°, 14.3°, 14.6°, 15.3°, 16.5°, 17.6°, 17.8°, 18.1°, 25.7°, 28.6°. The DSC spectrum had a characteristic peak at 123.9°C; the melting point of the hydrate product was 122.7°C, which was 50°C higher than the temperature of the amorphous desolventized product. Compared with the original amorphous product, the hydrate product had no pungent odor and no hygroscopicity.

The X-ray powder diffraction pattern of the crystal form of the valnemulin hydrochloride hydrate in Example 4 had characteristic peaks at diffraction angle 2θ = 9.3°, 9.5°, 10.4°, 11.7°, 12.3°, 12.6°, 12.7°, 14.4°, 14.5°, 15.3°, 16.6°, 17.6°, 17.9°, 18.1°, 25.6°, 28.7°. The DSC spectrum had a characteristic peak at 119.3°C; the melting point of the hydrate product was 119.3°C, which was 50°C higher than the temperature of the amorphous desolventized product. Compared with the original amorphous product, the hydrate product had no pungent odor and no hygroscopicity.

The X-ray powder diffraction pattern of the crystal form of the valnemulin hydrochloride hydrate in Example 5 had characteristic peaks at diffraction angle 2θ = 9.2°, 9.4°, 10.4°, 11.6°, 12.2°, 12.4°, 12.6°, 14.3°, 14.5°, 15.2°, 16.6°, 17.5°, 17.7°, 18.0°, 25.7°, 28.7°. The DSC spectrum had a characteristic peak at 122.2°C; the melting point of the hydrate product was 121.4°C, which was 50°C higher than the temperature of the amorphous desolventized product. Compared with the original amorphous product, the hydrate product had no pungent odor and no hygroscopicity.

The X-ray powder diffraction pattern of the crystal form of the valnemulin hydrochloride hydrate in Example 6 had characteristic peaks at diffraction angle 2θ = 9.3°, 9.5°, 10.4°, 11.8°, 12.3°, 12.7°, 14.5°, 14.6°, 15.1°, 16.6°, 17.7°, 18.0°, 18.3°, 25.9°, 28.9°. The DSC spectrum had a characteristic peak at 124.4°C; the melting point of the hydrate product was 123.4°C, which was 50°C higher than the temperature of the amorphous desolventized product. Compared with the original amorphous product, the hydrate product had no pungent odor and no hygroscopicity.

The X-ray powder diffraction pattern of the crystal form of the valnemulin hydrochloride hydrate in Example 7 had characteristic peaks at diffraction angle 2θ = 9.2°, 9.5°, 10.4°, 11.7°, 12.2°, 12.4°, 12.7°, 14.4°, 14.6°, 15.0°, 16.6°, 17.6°, 18.0°, 18.3°, 25.8°, 28.7°. The DSC spectrum had a characteristic peak at 124.9°C; the melting point of the hydrate product was 124.4°C, which was 50°C higher than the temperature of the amorphous desolventized product. Compared with the original amorphous product, the hydrate product had no pungent odor and no hygroscopicity.

### Comparative Example

A patented crystal form was obtained according to the crystallization method described in Example 1 of CN104876841B. The X-ray diffraction pattern of the obtained crystal form had characteristic peaks at diffraction angle 2θ = 8.4, 10.4, 10.8, 11.1, 12.0, 12.4, 14.8, 17.6, 18.2, 19.2, 20.1, 20.7, 25.7, 27.5, 28.4, 29.0.

### Example 8 Study on druggability of crystal form of hydrate

The effect of temperature (25 °C, 40 °C, and 60 °C), humidity (75% RH and 90% RH) and complexed condition 60 °C+75%RH on crystal forms was investigated by performing hygroscopicity experiments and influencing factor experiments on the valnemulin hydrochloride amorphous substance (having a content of 97.50% before placement, white solid), the crystal form substance of CN104876841B (the crystal form of Comparative Example) and the crystal form of the valnemulin hydrochloride hydrate in Example 1 of the present application (having a content of 99.25% before placement, white solid). The results are as follows.

**Table 1 Comparative study on stability of valnemulin hydrochloride hydrate, amorphous substance and the comparative crystal form**

| Placement conditions and time | Starting crystal form | Content after placement (%) | With or without hygroscopic ity | Appearance change | Crystal form change |
|---|---|---|---|---|---|
| 25 °C, 90% relative humidity, placed for 30 days | The crystal form of valnemulin hydrochloride hydrate (Example 1) | 99.23 | Without hygroscopic ity | White solid | No change |
| | The amorphous substance of valnemulin hydrochloride | 94.56 | Severe hygroscopic ity | Yellow transparent viscous solid | / |
| | The crystal form of Comparative Example | 99.21 | Slightly | White solid | Slight change |
| 40 °C, 75% relative humidity, placed for 30 days | The crystal form of valnemulin hydrochloride hydrate (Example 1) | 99.20 | Without hygroscopic ity | White solid | No change |
| | The amorphous substance of valnemulin hydrochloride | 90.82 | Severe hygroscopic ity | Yellow transparent viscous solid | / |
| | The crystal form of Comparative Example | 99.18 | Slightly | White solid | No change |
| 60 °C, 75% relative humidity, placed for 30 days | The crystal form of valnemulin hydrochloride hydrate (Example 1) | 98.78 | Without hygroscopic ity | White solid | No change |
| | The amorphous substance of valnemulin hydrochloride | 86.12 | Severe hygroscopic ity | Yellow | / |
| | The crystal form of Comparative Example | 98.69 | Slightly | Light yellow solid | No change |
| 60 °C, placed | The crystal form of | 98.47 | Without | White solid | No |
| in normal humidity for 30 days | valnemulin hydrochloride hydrate (Example 1) | | hygroscopic ity | | change |
| | The amorphous substance of valnemulin hydrochloride compound | 89.18 | Hygroscopi city | Yellow solid | / |
| | The crystal form of Comparative Example | 98.39 | Slightly | Light yellow solid | No change |

As can be seen from Table 1, the hydrate crystal was stable and the content did not change. While, the amorphous substance absorbed moisture severely, and the content decreased very seriously at high temperature, and the color changed. The crystal form of patent CN104876841B was slightly hygroscopic, and the color slightly changed at high temperature under high humidity.

The hydrate crystal and the amorphous substance and the crystal form of patent CN104876841B were compared in terms of bulk density, solubility, ect, as shown in the following table.

**Table 2 Physicochemical properties comparison of the valnemulin hydrochloride hydrate, the amorphous substance and the Comparative Example**

| API | Bulk density | Hygroscopicity | Solubility |
|---|---|---|---|
| The amorphous substance of valnemulin hydrochloride | 0.338 g/ml | 8.86% | 1 g/9 ml water |
| The crystal form of Comparative Example | 0.636 g/ml | 1.015% | 1 g/49 ml water |
| The crystal form of valnemulin hydrochloride hydrate (Example 1) | 0.596 g/ml | 0% | 1 g/28.9 ml water |

As can be seen from Table 2, the bulk density of the hydrate nearly doubled, and the irritation also decreased. It is more convenient and easy to operate than amorphous substances in API production and packaging. Further, it also brings convenience to the later preparation process operation. Although the solubility was lower than that of the amorphous form, it can fully meet the needs of clinical medication. Compared with the crystal form of CN104876841B, the crystal form of valnemulin hydrochloride hydrate was significantly better than the crystal form of CN104876841B in terms of hygroscopicity and solubility.

In summary, the crystal form of the valnemulin hydrochloride hydrate obtained in the present application has no obvious changes in content, appearance, crystal form, etc., and has good stability. Compared with the amorphous substance and the crystal form of patented CN104876841B, the hydrate has a better bulk density and no hygroscopicity, so it can be better applied to formulations.

The crystal form of the valnemulin hydrochloride hydrate and the preparation method proposed in the present application have been described in the examples. It should be understood that these descriptions are only to further illustrate the features and advantages of the present application, rather than limiting the claims of the present application.

## Claims

1. A crystal form of a valnemulin hydrochloride hydrate, having characteristic peaks at 2θ of 9.2±0.2°, 9.5 ± 0.2°, 10.3 ± 0.2°, 11.8 ± 0.2°, 12.3 ± 0.2°, 12.8 ± 0.2°, 15.1 ± 0.2°, 17.5 ± 0.2°, and 18.2 ± 0.2° in X-ray powder diffraction pattern of the crystal form of the hydrate as measured with CuKa rays;
wherein a moisture content of the crystal form of the hydrate is 0.1%-7%.

2. The crystal form of the valnemulin hydrochloride hydrate according to claim 1, further comprising characteristic peaks at one or more of 2θ of 4.4±0.2°, 12.6±0.2°, 14.4±0.2°, 14.6 ±0.2°, 16.5±0.2°, 17.9±0.2°, 18.7±0.2°, 25.8±0.2°, and 28.7±0.2° in X-ray powder diffraction pattern of the crystal form of the hydrate as measured with CuKa rays.

3. The crystal form of the valnemulin hydrochloride hydrate according to claim 1 or 2, wherein the DSC spectrum of the crystal form of the hydrate has an endothermic peak at 120±5°C.

4. A preparation method of the crystal form of the valnemulin hydrochloride hydrate according to any one of claims 1 to 3, comprising the following steps:
(1) thermally-dissolving valnemulin hydrochloride in a mixture of organic solvent/purified water at 20 °C to 60 °C;
(2) stirring under heat preservation for 0.5 h to 1 h, and cooling at -5 °C to 15 °C to precipitate a crystal from the mixed solution;
(3) filtering to obtain filter cake A;
(4) drying the filter cake A under vacuum at 20 °C to 40 °C to obtain filter cake B;
(5) stirring and equilibrating the filter cake A obtained in step (3) in a poor solvent for 6 h to 20 h, filtering, and drying under vacuum at 20 °C to 60 °C to obtain a hydrate crystal; or, humidifying the filter cake B obtained in step (4) at a humidity of 65% to 95% for 12 h to 48 h to obtain a hydrate crystal.

5. The preparation method according to claim 4, wherein the organic solvent is selected from one or more of ester, ketone, alcohol, furan or acetonitrile solvents; and the poor solvent is selected from one or more of alkane solvents, aromatic hydrocarbons, ether solvents or water.

6. The preparation method according to claim 5, wherein the organic solvent is selected from one or more of ethyl acetate, acetone, isopropanol, n-butanol, tert-butanol, methanol, ethanol, tetrahydrofuran or acetonitrile; and the poor solvent is selected from one or more of n-hexane, cyclohexane, n-heptane, diethyl ether, dimethyl ether, diisopropyl ether, methyl tert-butyl ether, toluene or water.

7. The preparation method according to claim 4, wherein the weight-volume ratio of valnemulin hydrochloride as an active pharmaceutical ingredient and the organic solvent/purified water is 1:1 to 1:10; the volume ratio of the organic solvent and purified water is 2:4 to 3:2; and the weight-volume ratio of valnemulin hydrochloride as an active pharmaceutical ingredient and the poor solvent is 1:2 to 1:15.

8. A pharmaceutical composition, containing the crystal form of the valnemulin hydrochloride hydrate according to any one of claims 1-3.

9. The pharmaceutical composition according to claim 8, wherein the pharmaceutical composition is a mixture of the crystal form of the valnemulin hydrochloride hydrate and another crystal form of valnemulin hydrochloride;
or, the pharmaceutical composition further contains one or more pharmaceutically acceptable carriers and/or excipients.

10. Use of the crystal form of the valnemulin hydrochloride hydrate according to any one of claims 1 to 3 or the pharmaceutical composition according to any one of claims 8 to 9 in the preparation of pharmaceutical preparations.

## Patentansprüche

1. Kristallform eines Valnemulin-Hydrochlorid-Hydrats mit charakteristischen Peaks bei 20 von 9,2 ± 0,2°, 9,5 ± 0,2°, 10,3 ± 0,2°, 11,8 ± 0,2°, 12,3 ± 0,2°, 12,8 ± 0,2°, 15,1 ± 0,2°, 17,5 ± 0,2° und 18,2 ± 0,2° im Röntgenpulverdiffraktionsmuster der Kristallform des Hydrats, gemessen mit CuKa-Strahlung;
wobei der Feuchtigkeitsgehalt der Kristallform des Hydrats 0,1 %-7 % beträgt.

2. Kristallform des Valnemulin-Hydrochlorid-Hydrats nach Anspruch 1, ferner umfassend charakteristische Peaks bei einem oder mehreren 2θ-Werten von 4,4 ± 0,2°, 12,6 ± 0,2°, 14,4 ± 0,2°, 14,6 ± 0,2°, 16,5 ± 0,2°, 17,9 ± 0,2°, 18,7 ± 0,2°, 25,8 ± 0,2° und 28,7 ± 0,2° im Röntgenpulverdiffraktionsmuster der Kristallform des Hydrats, gemessen mit CuKa-Strahlung.

3. Kristallform des Valnemulin-Hydrochlorid-Hydrats nach Anspruch 1 oder 2, wobei das DSC-Spektrum der Kristallform des Hydrats einen endothermen Peak bei 120 ± 5 °C aufweist.

4. Herstellungsverfahren der Kristallform des Valnemulin-Hydrochlorid-Hydrats nach einem der Ansprüche 1 bis 3, umfassend folgende Schritte:
(1) thermisches Auflösen von Valnemulin-Hydrochlorid in einem Gemisch aus organischem Lösungsmittel/Reinstwasser bei 20 °C bis 60 °C;
(2) Rühren unter Wärmeerhaltung für 0,5 h bis 1 h und Abkühlen auf -5 °C bis 15 °C, um einen Kristall aus der Mischlösung auszufällen;
(3) Filtrieren zur Gewinnung eines Filterkuchens A;
(4) Trocknen des Filterkuchens A unter Vakuum bei 20 °C bis 40 °C zur Gewinnung eines Filterkuchens B;
(5) Rühren und Äquilibrieren des in Schritt (3) erhaltenen Filterkuchens A in einem schlechten Lösungsmittel für 6 h bis 20 h, Filtrieren und Trocknen unter Vakuum bei 20 °C bis 60 °C zur Gewinnung eines Hydratkristalls; oder Befeuchten des in Schritt (4) erhaltenen Filterkuchens B bei einer Luftfeuchtigkeit von 65 % bis 95 % für 12 h bis 48 h zur Gewinnung eines Hydratkristalls.

5. Herstellungsverfahren nach Anspruch 4, wobei das organische Lösungsmittel aus einem oder mehreren der folgenden Lösungsmittel ausgewählt ist - Ester-, Keton-, Alkohol-, Furan- oder Acetonitrillösungsmittel - und das schlechte Lösungsmittel aus einem oder mehreren der folgenden Lösungsmittel ausgewählt ist - Alkansolventien, aromatische Kohlenwasserstoffe, Etherlösungsmittel oder Wasser.

6. Herstellungsverfahren nach Anspruch 5, wobei das organische Lösungsmittel aus einem oder mehreren der folgenden Lösungsmittel ausgewählt ist - Ethylacetat, Aceton, Isopropanol, n-Butanol, tert-Butanol, Methanol, Ethanol, Tetrahydrofuran oder Acetonitril - und das schlechte Lösungsmittel aus einem oder mehreren der folgenden Lösungsmittel ausgewählt ist - n-Hexan, Cyclohexan, n-Heptan, Diethylether, Dimethylether, Diisopropylether, Methyl-tert-Butylether, Toluol oder Wasser.

7. Herstellungsverfahren nach Anspruch 4, wobei das Gewichts-Volumen-Verhältnis von Valnemulin-Hydrochlorid als pharmazeutischem Wirkstoff zu dem organischen Lösungsmittel/Reinstwasser 1:1 bis 1:10 beträgt; das Volumenverhältnis des organischen Lösungsmittels zu Reinstwasser 2:4 bis 3:2 beträgt; und das Gewichts-Volumen-Verhältnis von Valnemulin-Hydrochlorid als pharmazeutischem Wirkstoff zu dem schlechten Lösungsmittel 1:2 bis 1:15 beträgt.

8. Pharmazeutische Zusammensetzung, umfassend die Kristallform des Valnemulin-Hydrochlorid-Hydrats nach einem der Ansprüche 1 bis 3.

9. Pharmazeutische Zusammensetzung nach Anspruch 8, wobei die pharmazeutische Zusammensetzung ein Gemisch aus der Kristallform des Valnemulin-Hydrochlorid-Hydrats und einer anderen Kristallform von Valnemulin-Hydrochlorid ist;
oder wobei die pharmazeutische Zusammensetzung ferner einen oder mehrere pharmazeutisch akzeptable Träger und/oder Hilfsstoffe umfasst.

10. Verwendung der Kristallform des Valnemulin-Hydrochlorid-Hydrats nach einem der Ansprüche 1 bis 3 oder der pharmazeutischen Zusammensetzung nach einem der Ansprüche 8 bis 9 zur Herstellung pharmazeutischer Zubereitungen.

## Revendications

1. Forme cristalline d'un hydrate de chlorhydrate de valnémuline, présentant des pics caractéristiques à 2θ de 9,2±0,2°, 9,5±0,2°, 10,3±0,2°, 11,8±0,2°, 12,3±0,2°, 12,8±0,2°, 15,1±0,2°, 17,5±0,2° et 18,2±0,2° dans un motif de diffraction de rayons X sur poudre de la forme cristalline de l'hydrate tel que mesuré avec des rayons CuKa;
dans laquelle la teneur en humidité de la forme cristalline de l'hydrate est de 0,1 %-7 %.

2. Forme cristalline de l'hydrate de chlorhydrate de valnémuline selon la revendication 1, comprenant en outre des pics caractéristiques à un ou plusieurs 2θ de 4,4±0,2°, 12,6±0,2°, 14,4±0,2°, 14,6±0,2°, 16,5±0,2°, 17,9±0,2°, 18,7±0,2°, 25,8±0,2° et 28,7±0,2° dans un motif de diffraction de rayons X sur poudre de la forme cristalline de l'hydrate tel que mesuré avec des rayons CuKa.

3. Forme cristalline de l'hydrate de chlorhydrate de valnémuline selon la revendication 1 ou 2, dans laquelle le spectre DSC de la forme cristalline de l'hydrate présente un pic endothermique à 120±5°C.

4. Procédé de préparation de la forme cristalline de l'hydrate de chlorhydrate de valnémuline selon l'une quelconque des revendications 1 à 3, comprenant les étapes suivantes :
(1) dissolution thermique du chlorhydrate de valnémuline dans un mélange de solvant organique/eau purifiée à 20°C à 60°C ;
(2) agitation sous conservation à chaud pendant 0,5 h à 1 h, puis refroidissement à -5°C à 15°C pour précipiter un cristal à partir de la solution mixte ;
(3) filtration pour obtenir un gâteau de filtration A ;
(4) séchage du gâteau de filtration A sous vide à 20°C à 40°C pour obtenir le gâteau de filtration B ;
(5) agitation et équilibrage du gâteau de filtration A obtenu à l'étape (3) dans un solvant pauvre pendant 6 h à 20 h, filtration et séchage sous vide à 20°C à 60°C pour obtenir un cristal d'hydrate ; ou humidification du gâteau de filtration B obtenu à l'étape (4) à une humidité de 65 % à 95 % pendant 12 h à 48 h pour obtenir un cristal d'hydrate.

5. Procédé de préparation selon la revendication 4, dans lequel le solvant organique est choisi parmi un ou plusieurs parmi les esters, les cétones, les alcools, les furanes ou les acétonitriles ; et le solvant pauvre est choisi parmi un ou plusieurs parmi les solvants alcanes, les hydrocarbures aromatiques, les solvants éthers ou l'eau.

6. Procédé de préparation selon la revendication 5, dans lequel le solvant organique est choisi parmi un ou plusieurs parmi l'acétate d'éthyle, l'acétone, l'isopropanol, le n-butanol, le tert-butanol, le méthanol, l'éthanol, le tétrahydrofurane ou l'acétonitrile ; et le solvant pauvre est choisi parmi un ou plusieurs parmi le n-hexane, le cyclohexane, le n-heptane, l'éther diéthylique, l'éther diméthylique, l'éther diisopropylique, le méthyl tert-butyl éther, le toluène ou l'eau.

7. Procédé de préparation selon la revendication 4, dans lequel le rapport poids-volume du chlorhydrate de valnémuline comme ingrédient pharmaceutique actif et du solvant organique/eau purifiée est de 1:1 à 1:10 ; le rapport volumique du solvant organique et de l'eau purifiée est de 2:4 à 3:2 ; et le rapport poids-volume du chlorhydrate de valnémuline comme ingrédient pharmaceutique actif et du solvant pauvre est de 1:2 à 1:15.

8. Composition pharmaceutique contenant la forme cristalline de l'hydrate de chlorhydrate de valnémuline selon l'une quelconque des revendications 1-3.

9. Composition pharmaceutique selon la revendication 8, dans laquelle la composition pharmaceutique est un mélange de la forme cristalline de l'hydrate de chlorhydrate de valnémuline et d'une autre forme cristalline de chlorhydrate de valnémuline ;
ou, la composition pharmaceutique contient en outre un ou plusieurs support(s) et/ou excipient(s) pharmaceutiquement acceptables.

10. Utilisation de la forme cristalline de l'hydrate de chlorhydrate de valnémuline selon l'une quelconque des revendications 1 à 3 ou de la composition pharmaceutique selon l'une quelconque des revendications 8 à 9 dans la préparation de préparations pharmaceutiques.
